Europäisches Patentamt

European Patent Office

Office européen des brevets

Publication number: **0 285 979**

**A2**

# EUROPEAN PATENT APPLICATION

Application number: 88105047.0

Int. Cl.4 **C12N 1/04**

Date of filing: **29.03.88**

Priority: **06.04.87 US 34632**

Date of publication of application:
**12.10.88 Bulletin 88/41**

Designated Contracting States:
**DE ES FR GB IT SE**

Applicant: **MILES INC.**
**1127 Myrtle Street**
**Elkhart Indiana 46514(US)**

Inventor: **Enders, George L., Jr.**
**22797 Bainbridge Drive**
**Elkhart IN 46514(US)**
Inventor: **Kamara, Bassie Jabron**
**234 Village Drive**
**Mishawaka IN 46545(US)**
Inventor: **Kim, Hyung Soo**
**10360 Hickory Hills Court**
**Osceola IN 46561(US)**

Representative: **Dänner, Klaus, Dr. et al**
**c/o Bayer AG Konzernverwaltung RP**
**Patentabteilung**
**D-5090 Leverkusen 1 Bayerwerk(DE)**

**Method for enhancing the stability of freeze-dried cultures.**

Disclosed is a method for stabilizing, i.e. enhancing the storage viability, of freeze-dried lactic acid producing bacteria. The method involves combining the bacteria with a mixture of cystine and a food grade bulking agent.

EP 0 285 979 A2

0 285 979

# METHOD FOR ENHANCING THE STABILITY OF FREEZE-DRIED CULTURES

## Background of the Invention

The present invention relates to the production of improved microbiological cultures and particularly to the production of dried, viable cultures of lactic acid producing bacteria (lactic bacteria) that will retain their viability at ambient or slightly reduced temperatures for extended periods of time.

The food industry employs the incorporation of viable lactic bacteria into a variety of food products for flavor texture development and preservation. Accordingly, there is a substantial demand for preserved lactic bacteria cultures that are simple to use, low in cost and easy to maintain. Preservation of these bacteria is commonly achieved by deep freezing or lyophilization. Deep freezing in liquid nitrogen is effective and relatively inexpensive. However, storage and transportation of the frozen cultures is complicated by the necessity of keeping them in the frozen state such as by the use of insulated carriers containing dry ice. Lyophilized cultures can be packaged in foil wrappers and stored at normal or only slightly reduced temperatures but undergo a reduction in viability (cell count) during the freeze-drying process with further losses in cell count during storage.

In order to maintain high cell counts in lyophilized cultures. cryoprotectants and stabilizers are typically added to the culture concentrate before freezing. For example Valdez et al in Cryobiology 20. 560-566 (1983) discuss the use of cysteine, asparagine and sodium glutamate as stabilizers for lactic bacteria during the freeze drying process. These compounds were added to the liquid culture concentrate prior to freeze-drying. More particularly, this reference reports the addition of the following solutions to the microorganism concentrate:

    a) 10% non-fat milk solids + 0.04% L-Cysteine
    b) 20% non-fat milk solids + 0.04% L-Cysteine
    c) Distilled water The pH of the solutions was adjusted to 6.8 - 7.0 with NaOH or HCl whereupon samples of the suspension were dispensed into ampoules and freeze-dried. These studies indicated that L-Cysteine exhibited cryoprotective properties during the freeze-drying step but made no mention of any beneficial effect on storage stability.

## Summary of the Invention

The present invention involves a method of increasing the viability, i.e. retention of viable cell count, of freeze-dried lactic acid producing bacteria which comprises combining the bacteria with a food grade bulking agent together with a stabilizing amount of cystine.

## Description of the Invention

Cysteine. $HSCH_2CH(NH_2)COOH$, is an amino acid which can be derived from the degradation of cystine, $HOOCCH(NH_2)CH_2SSCH_2CH(NH_2)COOH$. While cysteine has been shown to have some cryoprotective effect when introduced to a solution of lactic bacteria before freeze-drying, it has not been reported to provide a stabilizing effect when combined with the freeze-dried bacteria. Freeze-dried bacteria are normally combined with a bulking agent which serves the purpose of extending the volume of the culture concentrate or diluting its viability (CFU/gm) to a desired level. Suitable bulking agents include lactose, casein, non-fat milk solids, sweet whey, maltodextrin, xanthan gum, or combinations of these ingredients. In the practice of the present invention cystine can be added to the freeze-dried lactic bacteria either before or after it is mixed with the bulking agent or a mixture of the two can be added to the lactic bacteria preparation. In a preferred embodiment either the cystine is heated and then mixed with the bulking agent or the bulking agent and cystine are pre-mixed and then heated. The resulting mixture is then combined with the freeze-dried lactic bacteria. Such combination is typically achieved by blending the cystine-bulking agent mixture with the bacteria using a suitable dry ingredient blending device such as a Hobart mixer. In the preferred embodiment the bulking agent/cystine combination is typically heated to a temperature of from 60°C to 100°C for a period of from 5 to 20 minutes. The optional heating time will tend to decrease as the temperature is raised towards 100°C and vice-versa.

Lactic acid producing bacteria whose viability after freeze-drying can be enhanced by the present

2

method include *Streptococcus lactis. S. cremoris. S. diacetylactis. S. thermophilus. Lactobacillus bulgaricus. L. acidophilus. L. helveticus. L. bifidus. L. casei. L. lactis. L. plantarum. L. delbrueckii. L. fermentum. Pediococcus cerevisiae. P. acidilactici* and *Leuconostoc mesenteroides. Bifidobacterium bifidum. B. thermophilum.*

While the amount of cystine (either D. L or DL) is not critical, we prefer to use at least about 1.5% (w/w) of the combination of bulking agent and lactic bacteria being stabilized and the bulking agent with a maximum amount of about 5% (w/w) being typical. The weight ratio of lactic bacteria to bulking agent is typically from about 1:1 to 1:100.

The method of practicing the present invention is further illustrated by the following examples:

Example I

Five gram samples of milled, freeze dried *Lactobacillus acidophilus* were weighed out to be mixed into a dry blend with non-fat milk solids. One culture sample was homogeneously mixed with 45 grams of non-fat milk solids.

*L-cystine* (Signia Chemical Co.), 1.6 grams, was added to 43.5 grams of non-fat milk solids, heated at 100°C for 8 minutes and cooled to room temperature. Powder culture, 5.0 grams, was then added and mixed to obtain a homogeneous formulation containing 10% culture w/w. The mixtures were packaged in air using Naska polyethylene bags and stored at 4°C and 22°C for stability evaluation. Neither nitrogen flushing nor vacuum headspace were employed.

Enumeration of viable cells was determined by serial dilution of the mixture using sterile peptone water (0.1% w/v) and surface plating on Difco All Purpose Tween (APT) medium. Colony forming units per gram (CFU/gm) were counted after incubation for 48 hours at 37°C in a 10% carbon dioxide atmosphere. Only plates containing between 30 and 300 colonies were counted.

The storage results for the dry blends of culture with non-fat milk solids (NFMS) and non-fat milk solids treated with L-cystine are illustrated in Table I. A 1.8 fold improvement at 4°C upon 15 days storage and a 2.5 fold improvement after 7 days storage at 22°C are evident.

## TABLE I

### 4°C Storage

| DAYS | NFMS BLEND CFU/GM | % RECOVERY | NFMS + L-CYSTINE BLEND CFU/GM | % RECOVERY |
|---|---|---|---|---|
| 0 | $2.1 \times 10^{11}$ | - | $2.1 \times 10^{11}$ | - |
| 7 | $1.9 \times 10^{11}$ | 90.5 | $2.1 \times 10^{11}$ | 100 |
| 15 | $4.9 \times 10^{10}$ | 23.2 | $8.8 \times 10^{12}$ | 41.9 |

### 22°C Storage

| DAYS | NFMS BLEND CFU/GM | % RECOVERY | NFMS + L-CYSTINE BLEND CFU/GM | % RECOVERY |
|---|---|---|---|---|
| 0 | $2.1 \times 10^{11}$ | - | $2.1 \times 10^{11}$ | - |
| 7 | $2.4 \times 10^{10}$ | 11.4 | $6.0 \times 10^{10}$ | 28.6 |
| 10 | $5.3 \times 10^{9}$ | 2.5 | $7.4 \times 10^{9}$ | 3.5 |

## EXAMPLE II

The procedure of Example I was carried out with a freeze dried strain of *Bifidobacterium thermophilum*. In the enumeration of viable cells. Lactobacilli MRS medium was used: colonies were counted after 48 hours at 37°C in an anaerobic atmosphere. Storage results are set out in Table II. A 4 fold improvement was observed after 15 days storage at 4°C and a 3.5 fold improvement was observed after 15 days storage at 22°C as a result of L-cystine treatment. The results of this experiment are set out in Table II.

## TABLE II

### 4°C STORAGE

| | NFMS BLEND | | NFMS + L-CYSTINE BLEND | |
|---|---|---|---|---|
| DAYS | CFU/GM | % RECOVERY | CFU/GM | % RECOVERY |
| 0 | $7.0 \times 10^{11}$ | - | $7.0 \times 10^{11}$ | - |
| 7 | $3.2 \times 10^{11}$ | 46 | $9.8 \times 10^{11}$ | 100 |
| 10 | $1.0 \times 10^{11}$ | 14 | $6.1 \times 10^{11}$ | 87 |
| 15 | $6.6 \times 10^{10}$ | 9 | $2.5 \times 10^{11}$ | 36 |

### 22°C STORAGE

| | NFMS BLEND | | NFMS + L-CYSTINE BLEND | |
|---|---|---|---|---|
| DAYS | CFU/GM | % RECOVERY | CFU/GM | % RECOVERY |
| 0 | $7.0 \times 10^{11}$ | - | $7.0 \times 10^{11}$ | - |
| 7 | $4.6 \times 10^{10}$ | 6.6 | $9.8 \times 10^{10}$ | 14 |
| 10 | $7.6 \times 10^{10}$ | 11.0 | $1.2 \times 10^{11}$ | 17 |
| 15 | $2.8 \times 10^{10}$ | 4.0 | $9.8 \times 10^{10}$ | 14 |

## Example III

Three milled, freeze-dried cultures (*Pedicoccus acidilactici, Lactobacillus curvatus* and *Streptococcus faecium*) were weighed out based on specific activity to give a viability ratio of 1:1:1 and then mixed into a dry blend with non-fat milk solids. Each weighed culture sample was homogeneously mixed with non-fat milk solids to obtain approximately $6 \times 10^{10}$ total CFU/gm.

Heated L-cystine and non-fat milk solids were blended and the culture was formulated following the procedure described in Example I. In the enumeration of viable cells, Lactobacilli MRS medium was used: colonies were counted after 48 hours incubation at 37°C in a 10% carbon dioxide atmosphere.

The storage stability results for the dry blends of the three cultures with both non-fat milk solids and non-fat milk solids plus L-cystine are illustrated by Table III. From this table, it can be determined that as a result of L-cystine treatment, a 1.8 fold improvement was observed after 60 days storage at 4°C and a 2.5 fold improvement was observed after 60 days storage at 22°C.

## TABLE III

### 4°C Storage

| | NFMS BLEND | | NFMS + L-CYSTINE BLEND | |
|---|---|---|---|---|
| DAYS | CFU/GM | % RECOVERY | CFU/GM | % RECOVERY |
| 0 | $6.10 \times 10^{10}$ | -- | $6.13 \times 10^{10}$ | -- |
| 30 | $3.65 \times 10^{10}$ | 60 | $5.40 \times 10^{10}$ | 89 |
| 60 | $2.89 \times 10^{10}$ | 47 | $5.18 \times 10^{10}$ | 85 |

### 22°C Storage

| | NFMS BLEND | | NFMS + L-CYSTINE BLEND | |
|---|---|---|---|---|
| DAYS | CFU/GM | % RECOVERY | CFU/GM | % RECOVERY |
| 0 | $6.10 \times 10^{10}$ | -- | $6.13 \times 10^{10}$ | -- |
| 30 | $4.10 \times 10^{10}$ | 67 | $4.95 \times 10^{10}$ | 81 |
| 60 | $1.76 \times 10^{10}$ | 29 | $4.40 \times 10^{10}$ | 72 |

**Claims**

1. A method for increasing the viability of freeze-dried lactic acid producing bacteria culture concentrates which comprises combining the bacteria with a food grade bulking agent together with a stabilizing amount of cystine.

2. The method of claim 1 wherein the bulking agent is lactose, non-fat milk solids, casein, sweet whey, maltodextrin, xanthan gum or a combination thereof.

3. The method of claims 1 or 2 wherein the cystine is heated before it is combined with the lactic acid producing bacteria.

4. The method of claims 1 - 3 wherein the cystine is combined with the bulking agent and then heated.

5. The method of claim 4 wherein the bulking agent/cystine combination is heated to a temperature of from 60° to 100°C for a period of from 5 to 20 minutes.

6. The method of claims 1 - 5 wherein the amount of cystine is at least about 1.5 to 5 % (w/w) based on the combination of lactic acid producing bacteria and the bulking agent.

7. The method of claims 1 - 6 wherein the lactic acid producing bacteria is selected from the groups of *Streptococcus lactis, S. cremoris, S. diacetylactis, S. thermophilus, Lactobacillus bulgaricus, L. acidophilus, L. helveticus, L. bifidus, L. casei, L. lactis, L. plantarum, L. delbrueckii, L. fermentum, Pedicoccus cerevisiae, P. acidilactici* and *Leuconostoc mesenteroides, Bifidobacterium bifidum, B. thermophilum*.

8. A composition of matter comprising viable cells of a lactic acid producing bacteria, cystine and a bulking agent.

9. The composition of claim 8 wherein the cystine is present in an amount of from about 1.5 to 5% (w/w) of the combination of bulking agent and lactic acid producing bacteria and the weight ratio of the bacteria to the bulking agent is from about 1:1 to 1:100.

10. A method for increasing the viability of lactic acid producing bacteria culture concentrates which comprises the steps of:

a) Combining L-cystine with non-fat milk solids in a weight ratio of from 1:1 to 1:100;

5

b) heating the combination formed in step a to a temperature of from about 60° to 100°C for a period of from about 5 to 20 minutes; and

c) combining the combination heated in step b with the culture concentrate in such proportions that the weight ratio of the culture concentrate to non-fat milk solids is from 1:1 to 1:100 and the cvstine is present in an amount of from 1.5 to 5% (w·w) of the combination of non-fat milk solids and culture concentrate.